# EUROPEAN PATENT SPECIFICATION

(11) **EP 3 455 757 B1**
(45) Date of publication and mention of the grant of the patent: **25.08.2021**
(21) Application number: 17729018.6
(22) Date of filing: 08.05.2017
(51) Int. Cl.: G16H 50/80

(54) **METHOD, COMMUNICATION SYSTEM AND COMPUTER PROGRAM FOR PROVIDING INFORMATION INDICATIVE OF CONCENTRATION OF ALLERGENS IN THE ENVIRONMENT**
VERFAHREN, KOMMUNIKATIONSSYSTEM UND COMPUTERPROGRAMM ZUR BEREITSTELLUNG VON INFORMATIONEN, DIE AUF DIE KONZENTRATION VON ALLERGENEN IN DER UMGEBUNG HINDEUTEN
PROCÉDÉ, SYSTÈME DE COMMUNICATION ET PROGRAMME INFORMATIQUE PERMETTANT DE FOURNIR DES INFORMATIONS INDICATIVES SUR LA CONCENTRATION D'ALLERGÈNES DANS L'ENVIRONNEMENT

(30) Priority: 11.05.2016 IT UA20163359
(43) Date of publication of application: 20.03.2019
(73) Proprietor: Zambon S.p.A., 20091 Bresso MI (IT)
(72) Inventor: MUZIO, Massimiliano, 20091 Bresso (MI) (IT)
(74) Representative: Pietra, Giulia
(86) International application number: PCT/EP2017/060881
(87) International publication number: WO 2017/194447

(56) References cited:
- WO-A1-2014/207629
- WO-A2-2013/019368
- US-A1- 2010 318 424
- US-A1- 2015 242 586

## Description

The present invention relates in general to the field of apparatus and methods for processing digital data. In particular, the present invention relates to a method for providing information indicative of concentration of allergens in the environment and to a communication system and a computer program configured to implement said method.

According to the present invention, the expression "respiratory difficulty symptoms" comprises symptoms associated mainly with allergic rhinitis such as a sensation of blocked nasal respiration, runny nose (colorless, fluid, serous secretion emitted by the nasal mucous glands), itchiness of the nose which often affects the palate as well, and sneezing fits; and symptoms associated mainly with allergic asthma such as, wheezing, dyspnea (breathing difficulties), sensation of blocked chest and coughing.

In-depth studies have shown that there exists a close correlation between rhinitis and allergic asthma. It is estimated in fact that about one third of patients with rhinitis also suffer from asthma and that rhinitis is present in a very large number of persons suffering from allergic asthma. Precisely for these reasons, sometimes rhinitis and asthma are grouped together - practically as the same disorder - under the name "united airways disease" (UAD).

The aforementioned respiratory difficulty symptoms are usually triggered by the presence in the environment of allergens, such as pollens, which enter the organism via the respiration and trigger an anomalous immunological response.

The concentration of various types of pollens in the environment is typically detected by means of a network of particle measurement devices which are distributed over the territory. The data supplied by the particle measurement devices are collected in a pollen bulletin which is published (for example on an Internet site) so that it can be consulted by users. A pollen bulletin generally comprises a list of areas (for example in the form of an interactive map divided into regions) and, for each area, an indication of the average concentration (for example, none, low, medium or high) for each type of pollen.

A person suffering from allergic rhinitis and/or allergic asthma may consult the pollen bulletin selecting an area which is of interest to them and viewing the average concentration of the various types of pollens in the selected area. The person may thus evaluate in advance whether, if she/he decides to go to that area, she/he will be exposed to the risk of developing one or more of the symptoms associated with allergic rhinitis and/or asthma allergy and, if this is the case, may decide what measures to take (for example she/he may take with her/him a drug for counteracting the symptoms or may decide not to go to that area).

The Applicant has observed that the pollen bulletins obtained from the data detected by the particle measurement devices are not entirely reliable.

Said data is in fact updated only periodically, typically once a week. It therefore does not take into account variations in the concentrations of the pollens over shorter time scale, for example due to sudden variations in the weather conditions.

The pollen bulletins moreover provide the average concentration of the pollens in relatively large areas, typically regions. However, the concentration of an allergen may vary significantly from one place to another within the area. It may happen, for example that the average concentration of an allergen in a certain area as indicated by the bulletin is significantly lower than its actual concentration in the exact location where the person wishes to go. This may result, in a disadvantageous manner, in the person being exposed unknowingly to the risk of an allergic asthma and/or allergic rhinitis attack.

US 2015/242586 describes a system and method for presenting real-time health information which administers virtual questionnaires, automatically determines a user health status, and presents a visualization to a user indicating a likelihood of a symptom occurrence.

An object of the present invention is to provide a method for providing information indicative of concentration of allergens in the environment, which solves at least one of the aforementioned problems.

In particular, an object of the present invention is to provide a method for providing information indicative of concentration of allergens in the environment, which allows providing persons suffering from respiratory difficulty symptoms (associated generally with allergic asthma and/or rhinitis attacks) with more accurate information about the actual concentration of the allergens compared to that indicated in the pollen bulletins, so as to minimize the risk that these persons are exposed unknowingly to an allergic asthma and/or allergic rhinitis attack.

According to the present invention, a person detecting a respiratory difficulty symptom (presumably due to the presence of an allergen in the environment) records information relating to the detected symptom using her/his mobile device. The recording comprises providing subjective information relating to the detected respiratory difficulty symptom via a graphical interface of the mobile device. The subjective information comprises an indication of a probable cause of the detected respiratory difficulty symptom and an indication of the severity of the detected respiratory difficulty symptom. The mobile device completes the information relating to the detected symptom with automatically generated objective information specifying the place where the symptom was detected and date and time at which the symptom was detected. Then, at least part of the recorded information relating to the detected symptom is shared by the mobile device on a sharing server. Any other mobile device may receive the share part of the recorded information relating to the detected symptom from the sharing server and display it on a map shown by its display.

Therefore, basically, according to the present invention the information from the pollen bulletins as displayed by the mobile terminals of persons suffering from respiratory difficulty symptoms are corrected or completed by the shared information which those who have detected respiratory difficulty symptoms have recorded on their own mobile terminals.

This advantageously results in the provision of more accurate information about the actual concentration of the allergens compared to that indicated in the pollen bulletins, which allows to minimize the risk that a person suffering from respiratory difficulty symptoms is exposed unknowingly to an allergic asthma and/or allergic rhinitis attack.

First of all, indeed, the recorded information on the detected respiratory difficulty symptoms are particularly reliable. By interacting with the graphical interface of her/his mobile device (which the person always has with them), the person may indeed easily record on her/his mobile device the subjective and objective information relating to each respiratory difficulty symptom as soon as the event has finished. The information relating to each detected symptom is therefore advantageously complete and reliable, both because the person immediately provides subjective information (without therefore having to try to remember things days or weeks later) and because said subjective information is completed with objective information generated automatically by the mobile device.

Moreover, the recorded information on the detected respiratory difficulty symptoms are more accurate than those coming from the pollen bulletin, both from the spatial point of view and from the temporal point of view. Such information (and in particular the objective information) may indeed specify the exact location where the symptom has been detected, by contrast with the relatively large areas for which pollen bulletins usually provide average concentration of pollens. Besides, such information may be recorded and shared substantially in real time after the detected event is finished. Hence, variations in the concentrations of the pollens over very short time scale (for example due to sudden variations in the weather conditions) may be reflected in such information.

If, for example, a person has respiratory difficulty symptoms (for example symptoms of an asthma attack) which are very severe, presumably triggered by oleaceae plants in a location situated in an area which, according to the bulletin, should have had a low concentration of this type of pollen, the person may share with other persons at least some of the information relating to her/his symptoms recorded on her/his mobile device. In this way, the other persons are advantageously informed of the fact that, for example, the concentration of oleaceae plants in that location is presumably higher than that indicated in the bulletin and they may therefore avoid any accidental exposure to said allergen.

The invention is defined by the appended independent claims 1, 11 and 12. Optional aspects are provided by the dependent claims.

The present invention will be illustrated in greater detail by means of the attached drawings which are provided by way of a non-limiting 20 example and in which:
- Figure 1 schematically shows a communication system for providing information indicative of the concentration of allergens in the environment, according to an embodiment of the present invention;
- Figure 2 shows the main screen shown on the display of a mobile device of the communication system of Figure 1, according to an embodiment of the present invention;
- Figure 3 is a flow chart of the operation of the mobile device of Figure 2, according to an embodiment of the present invention;
- Figure 4 shows the screen of Figure 2, in which the pollen bulletin has been opened;
- Figure 5 shows the menu screen shown on the display of the mobile device according to an embodiment of the present invention;
- Figure 6 shows the recording screen shown on the display of the mobile device according to an embodiment of the present invention;
- Figure 7 shows the notifications screen shown on the display of the mobile device according to an embodiment of the present invention; and
- Figure 8 shows the screen of the diary shown on the display of the mobile device according to an embodiment of the present invention;

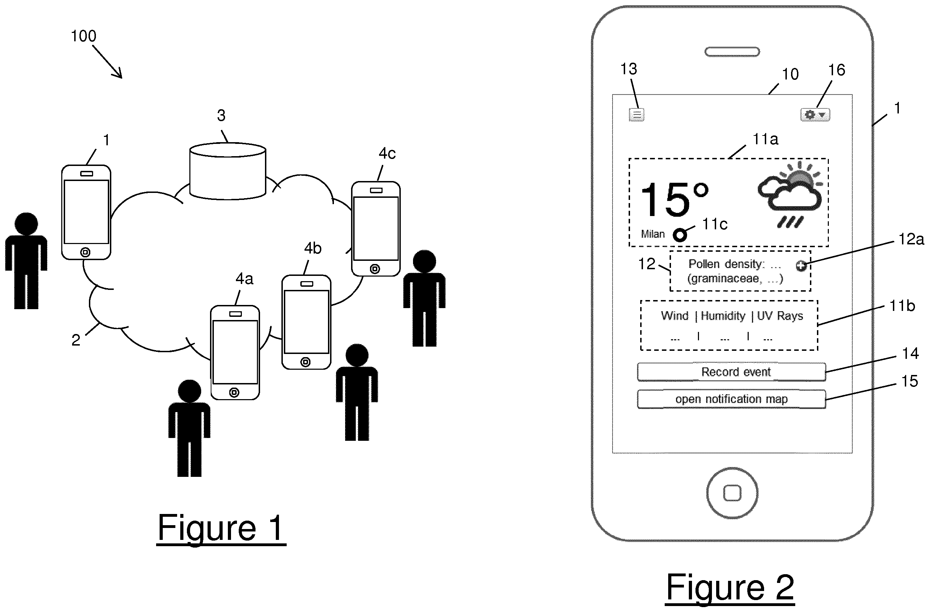

Figure 1 schematically shows a communication system 100 for providing information indicative of the concentration of allergens in the environment to a plurality of mobile devices, according to an embodiment of the present invention.

The communication system 100 comprises a mobile device 1, a mobile radio network 2, a sharing server 3 and a plurality of other mobile devices 4a, 4b, 4c. The mobile device 1 is preferably connected to the sharing server 3 by means of the mobile radio network 2, to which the mobile devices 4a, 4b, 4c are also connected.

The mobile devices 1, 4a, 4b and 4c preferably are portable devices able to connect autonomously to a mobile radio network, such as a GSM (Global System for Mobile communications) network, UMTS (Universal Mobile Telecommunication System) network, LTE (Long Term Evolution) network, etc. According to preferred embodiments, the mobile devices 1, 4a, 4b, 4c are smartphones or tablets provided with mobile radio connectivity.

The mobile devices 1, 4a, 4b and 4c are moreover preferably provided with geolocalization modules (not visible in the drawings) able to detect automatically the position of the respective mobile device 1, 4a, 4b, 4c. According to a preferred embodiment, the localization modules of the mobile devices 1, 4a, 4b, 4c are GPS (Global Positioning System) receivers which - as is known - are able to be located by a satellite network and to receive from it its own geographical coordinates.

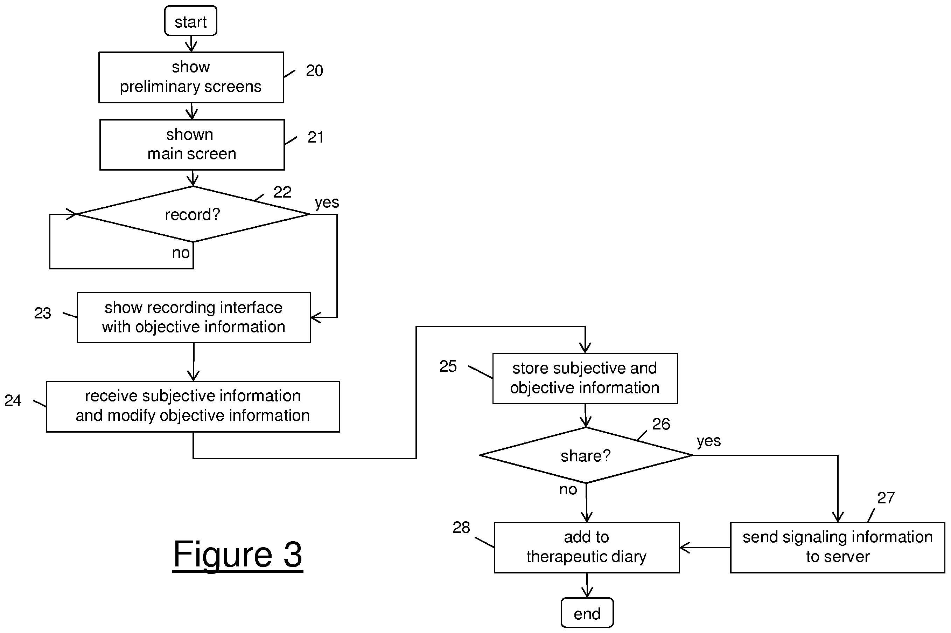

The mobile device 1, schematically depicted in Figure 2, is preferably provided with a software component (not shown in the drawings) which allows the recording of information relating to the detection of respiratory difficulty symptoms of a person. The operation of the software component is described in detail hereinbelow, with reference to the flow chart in Figure 3.

When the software component of the mobile device 1 is started by the person using the mobile device 1 for the first time (for example by selecting a suitable icon displayed on the display of her/his mobile device 1), preferably a sequence of preliminary screens, which are not shown in the drawings, is shown (step 20). These preliminary screens preferably comprise:
- a preliminary terms and conditions screen, which informs the person of the terms and conditions for use of the software component, which the person must accept before being able to use the software component;
- a preliminary informative screen, which provides a brief description of the functions of the software component (which will be described in detail hereinbelow);
- a preliminary profile screen, by means of which the person may provide certain personal data, such as sex, date of birth, whether allergic asthma and/or allergic rhinitis have already been diagnosed by a doctor and, if so, whether a specific therapy has been prescribed by the doctor. Preferably this personal data is stored only locally in the mobile device 1 and is not accessible remotely or made available via the mobile radio network to which the mobile device 1 is connected;
- a preliminary test screen where the person is asked a number of questions such that it is possible to obtain a summary indication of the level of severity of her/his allergic asthma and/or her/his allergic rhinitis. The test may for example be in accordance with the international GINA (Global Initiative for Asthma) 2014 guidelines. The outcome of the test is also stored only locally on the mobile device 1 and is not accessible remotely nor made available via the mobile radio network to which the mobile device 1 is connected.

After the preliminary screens have been displayed, a main screen 10, shown in Figure 2, is preferably shown on the display of the mobile device 1 (step 21).

The main screen 10 preferably comprises a weather window showing the name of a location and the weather information for that location, such as the temperature, weather conditions, wind, humidity, showers, UV rays, etc. For example, in the embodiment shown in Figure 2, the weather window is divided up into two sub-windows 11a (comprising name, location, temperature and weather situation) and 11b (comprising wind, humidity and UV rays). The location indicated in the weather window may be that of the mobile device 1 as automatically detected by its geolocalization module indicated above. Alternatively, the location indicated in the weather window 11 may be selected by the person who uses the mobile device 1 by selecting a suitable icon 11c, which opens a list of selectable locations.

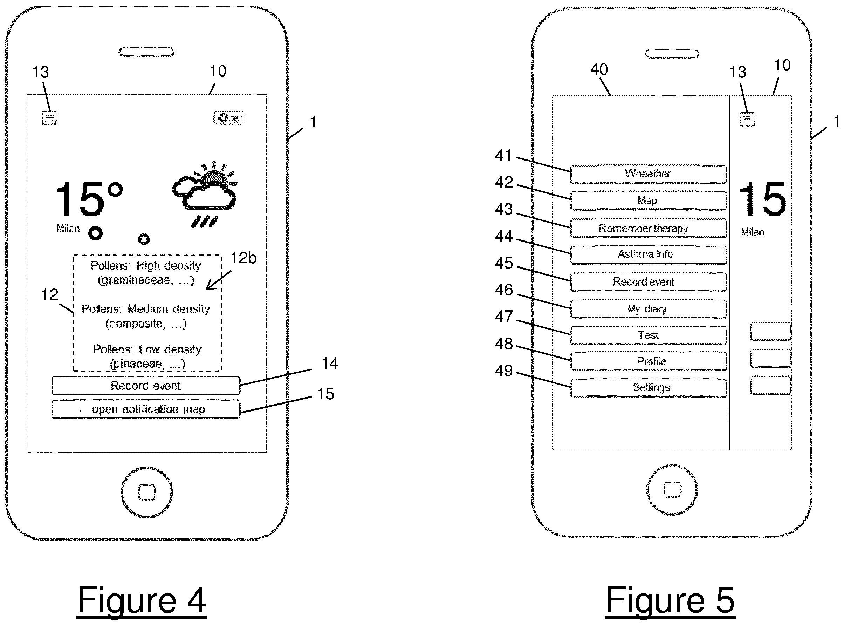

The main screen 10 preferably also comprises a pollen window 12 which comprises an icon 12a which, once selected, expands the pollen window 12 so as to show a pollen bulletin 12b, as shown in Figure 4. The pollen bulletin 12b preferably comprises indications of average concentrations of the various types of pollens in the geographical area (for example the region) which includes the location indicated in the weather window 11.

The weather information shown in the weather window and the average concentrations shown in the pollen bulletin 12b are preferably obtained from a weather forecast and environmental monitoring service, such as 3Bmeteo, Accuweather, etc.

The main screen 10 also comprises a menu icon 13 which, if selected, opens a menu screen 40 schematically shown in Figure 5. The menu screen 40 comprises a plurality of keys, each of which corresponds to a respective item of the menu. In particular, the menu screen 40 preferably comprises at least one of:
- a first key 41 which allows one to return to the main screen 10;
- a second key 42 which opens a map, as will be described in greater detail hereinbelow;
- a third key 43 which opens a graphical interface (not shown in the drawings) by means of which the person may set alarms for the date and the times when drugs must be taken (not necessarily for the treatment of allergic asthma and/or allergic rhinitis);
- a fourth key 44 which opens an information screen (not shown in the drawings) containing general information relating to the pathology of allergic asthma and/or allergic rhinitis, their causes, their symptoms, etc.;
- a fifth key 45 which opens a recording graphical interface, as described in greater detail hereinbelow;
- a sixth key 46 which allows access to a therapeutic diary, as will be described in greater detail hereinbelow;
- a seventh key 47 which allows the person to repeat the abovementioned test in order to obtain a summary indication of the degree of severity of her/his allergic asthma and/or her/his allergic rhinitis;
- an eighth key 48 which allows the person to access her/his profile as created upon start-up of the software component and, optionally, to modify it; and
- a ninth key 49 which allows the person to access the settings of the software component (for example password setting for access to the profile and/or the therapeutic diary).

Some of these keys, for greater convenience, are also present on the main screen 10 so as to allow the person using the mobile device 1 to access directly the corresponding function of the software component. In particular, returning to the main screen 10 shown in Figure 2, it preferably also comprises:
- a key 14 which, similar to the key 45 of the menu screen 40, opens a recording graphical interface, as will be described in greater detail hereinbelow;
- a key 15 which, similar to the key 42 of the menu screen 40, opens a map, as will be described in greater detail hereinbelow; and
- a key 16 which, similar to the key 49 of the main screen 40, allows the person to access the settings of the software component (for example password setting for access to the profile and/or the therapeutic diary).

Considering again the flow chart of Figure 3, according to embodiments of the present invention, at the end of detection of a respiratory difficulty symptom (for example an allergic rhinitis and/or allergic asthma symptom), the software component preferably allows the person to record on the mobile device 1 information relating to the respiratory difficulty symptom detected.

In particular, the software component preferably determines that the person wishes to record information relating to the detection of a respiratory difficulty symptom when the person presses the key 14 on the main screen 10 or, alternatively, the key 45 on the menu screen 40 (step 22).

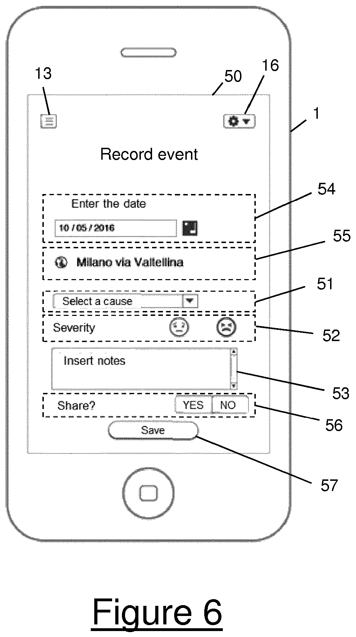

When the person presses the key 14 or 45, preferably a recording graphical interface 50, schematically shown in Figure 6, is shown on the display of the mobile device 1.

The recording graphical interface 50 is preferably configured to allow the person to enter subjective information relating to the detected respiratory difficulty symptom, namely information obtained according to the perception of the person suffering the symptom. This subjective information preferably comprises the probable cause (namely the type of allergen) which triggered the onset of the symptom (which the person may deduce for example by observing the type of trees present in the surrounding environment) and/or the severity of the symptom. For this purpose, according to a preferred variant, the recording graphical interface 50 comprises:
- a pull-down menu 51 which comprises a list of allergens which may be selected by the person, and
- a symptom severity selection window 52, which comprises icons (two for example) which indicate intuitively respective levels of severity of the symptom (slight or severe, for example).

The recording graphical interface 50 may also comprise a window 53 where the person may add notes about detection of the respiratory difficulty symptom, for example the activity which she/he was performing when the symptom occurred, any drugs taken to counteract the symptom, etc.

The recording graphical interface 50 is moreover preferably configured to display objective information relating to the detected respiratory difficulty symptom, generated automatically by the mobile device 1. This objective information preferably comprises the location where the symptom was detected and the date and time when the symptom was detected. For this purpose, according to a preferred variant, the recording graphical interface 50 comprises:
- a window for displaying and modifying the date and time 54, where the date and time as detected by the mobile device 1 via the mobile radio network are shown, and
- a window for displaying and modifying the location 55, where the current position of the mobile device 1 as detected by its geolocalization module is shown.

Preferably, both the date and the time shown in the window 54 and the location shown in the window 55 can be modified by the person, for example in the case where the recording is made a few hours or days after the disappearance of the symptom and/or after leaving the location where the symptom was detected.

The recording graphical interface 50 also comprises a sharing key 56, the function of which will be explained in greater detail hereinbelow. The recording graphical interface 50 also preferably comprises a save key 57 which allows the subjective and objective information described above to be saved locally.

Considering again the flow chart in Figure 3, once the recording graphical interface 50 has been displayed, the software component preferably receives the subjective information provided by the person via the pull-down menu 51 and the symptom severity selection window 52 of the recording graphical interface (step 24). During step 24, the software component preferably receives also any modifications to the objective information made by the person via the windows 53 and 54 of the recording graphical interface 50.

The objective and subjective information relating to the detection of the respiratory difficulty symptom of the person is then preferably stored locally on the mobile device 1 (step 25). Storage is preferably started by the person, by pressing the save key 57.

Advantageously, therefore, by interacting via the recording graphical interface 50 of her/his mobile device 1 (which the person always has with her/his), the person may easily record on her/his mobile device 1 the subjective information and objective information relating to each respiratory difficulty event as soon as the event has finished. The information relating to each difficulty event is therefore advantageously complete and reliable, both because the person immediately provides subjective information (without therefore having to try to remember things days or weeks later) and because said subjective information is completed with objective information generated automatically by the said mobile device 1.

Considering again the flow chart shown in Figure 3, while recording the subjective and objective information relating to the difficulty symptom detected in step 25, the person may decide to send out a notification, namely share part of this information (called below "signaling information") with the users of the mobile devices 4a, 4b, 4c, which preferably have similar software components running thereon. In order to start notification or sharing, the person preferably selects the key 56 present on the recording graphical interface 50.

When the software component, at the end of the recording step 25, determines that the key 56 has been selected and that therefore the person wishes to send a notification, the person preferably extracts the signaling information from the subjective and objective information recorded and sends it to a sharing sever 3 shown in Figure 1 (step 27).

As described above, the mobile device 1 is connected to the sharing server 3 by means of the mobile radio network 2 to which the mobile devices 4a, 4b, 4c of other persons, also provided with software components similar to that of the mobile device 1, are also connected.

Preferably, during the step 27, the software component of the mobile device 1 sends to the sharing server 3 signaling information comprising for example the place, the date and the allergen which triggered the onset of the detected respiratory difficulty symptom and, optionally, the severity of the symptom detected.

The signaling information are preferably stored on the sharing server 3 in an anonymous and non-traceable manner so that it is not possible to establish any connection between said information and the mobile device 1 from which it was sent. In this way, advantageously the privacy of the person is protected. The signaling information are moreover stored on the sharing server 3 for a finite time, for example 24 hours, at the end of which they are deleted.

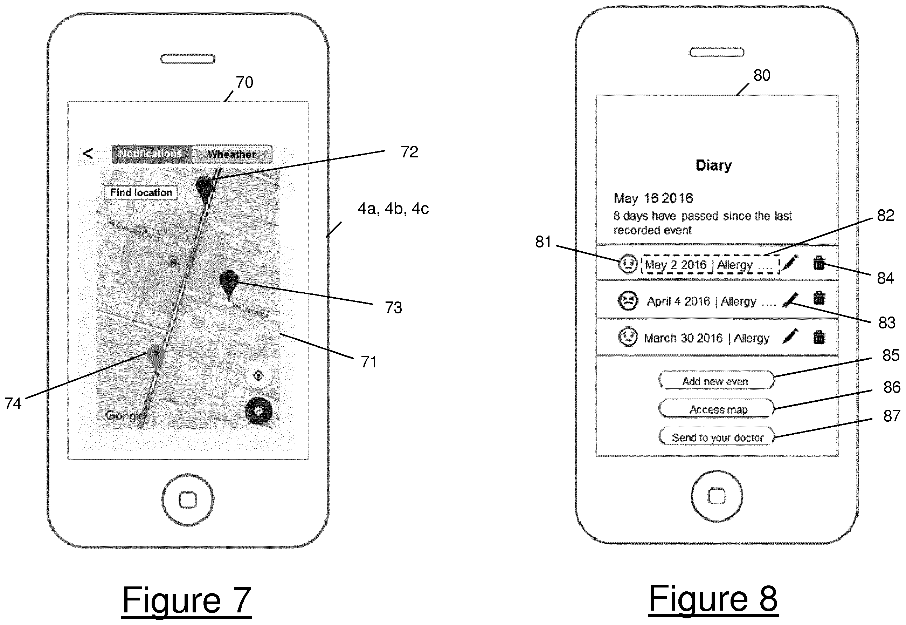

The notifications advantageously may be displayed by the mobile devices 4a, 4b, 4c of other persons, also provided with software components similar to that of the mobile device 1. In particular, the person who uses any one of the mobile devices 4a, 4b, 4c may select the key 15 on the main screen (or the key 42 on the menu screen 40) shown by the display of her/his mobile device. The selection of any one of these two keys opens a notifications screen 70, shown schematically in Figure 7.

The notifications screen 70 preferably shows a map 71. If the geolocalization device is active, the map 71 shows the locality where the mobile device 4a, 4b or 4c is situated. Otherwise, the map can be preferably navigated by means of a scroll function. The map 71 preferably shows the notifications sent by other persons relating to the respiratory difficulty symptoms detected in the vicinity of the locality where the mobile device 4a, 4b or 4c is situated. For example, the map 71 shown by way of example in Figure 7 shows three indicator symbols 72, 73 and 74, each of which represents a respective notification. For example, the indicator symbol 72 may correspond to a notification sent by the person who is using the mobile device 1 while carrying out step 27 of the flow diagram shown in Figure 3. Preferably, when an indicator symbol 72, 73 or 74 is selected, a pop-up window containing all the signaling information is opened.

The notifications relating to the detection of respiratory difficulty symptoms, shared by the person using the mobile device 1 and by other persons using the mobile devices 4a, 4b and 4c advantageously allow the information contained in the pollen bulletin 12b to be completed or corrected. If, for example, the person using the mobile device 1 detects a respiratory difficulty symptom (for example, one or more symptoms of an asthma attack) which is very severe, presumably triggered by oleaceae plants in a location situated in an area which, according to the pollen bulletin 12b, should have had a low concentration of this type of pollen, the person may decide to notify the persons who use the mobile devices 4a, 4b and 4c about the detection of the symptom. In this way the persons who use the mobile devices 4a, 4b and 4c are advantageously informed of the fact that the concentration of oleaceae plants in that location is presumably higher than that indicated by the pollen bulletin 12b.

Preferably, the exchange of signaling information between sharing server 3 and mobile devices 1, 4a, 4b, 4c is performed by means of connections managed by a cryptographic protocol which allows secure communication between source and recipient (end-to-end principle) on TCP/IP networks (such as the Internet) and which provides functions for authentication, data integrity and encryption when operating above the transportation level. For example, the exchange of signaling information may be performed using the SSL (Secure Socket Layer) certificate. Moreover, preferably, before being transmitted the signaling information are preferably encrypted, for example using the known Triple DES (Data Encryption Standard) algorithm.

The sharing server 3 is preferably implemented using Cloud Server technology, namely is implemented as a set of real servers physically located at the data center of the service provider. Cloud Server technology advantageously ensures a high degree of reliability (if a real server should stop, the system hardware allows the service to be kept active) and scalability (a given storage capacity and bandwidth may be initially used and amplified subsequently). For example, the sharing server 3 may be implemented on N+1 redundant multiprocessor machines with high-availability SAN (storage area network). All the Cloud Server systems are preferably provided with complete control of the Cloud Server via the Web (start-up, switch-off, monitoring), IPv4 and IPv6 dedicated network, dedicated virtual environment without overbooking, storage based on SAN and 99.90% guaranteed UpTime.

Considering again the flow chart shown in Figure 3, once locally stored during step 25, the subjective and objective information relating to the detected symptom is preferably entered automatically in a therapeutic diary which is also locally stored on the mobile device (step 28). This therapeutic diary is essentially a temporally ordered list of all the respiratory difficulty symptoms detected by the person and recorded on the mobile device 1. The recording of one detection after another on the mobile device 1 advantageously results therefore in the automatic compilation of a complete and reliable therapeutic diary which will allow the doctor to establish the most suitable therapy.

The therapeutic diary may be consulted by the person on the mobile device 1, for example by selecting the key 46 on the menu screen 40 shown in Figure 5. Preferably, when the person selects the key 46, the software component asks them for a password which the person may enter via the settings menu accessible by means of the key 16 of the main screen 10. According to a preferred variant, the software component may envisage a security mechanism with secret question, should the person not remember the password for accessing the diary. Once the person has accessed her/his therapeutic diary, it is shown on the display of the mobile device 1 in a diary screen 80 shown in Figure 8.

As shown in Figure 8, the diary screen 80 preferably comprises a temporally ordered list of the respiratory difficulty symptoms detected by the person and recorded on the mobile device 1. For each symptom detected, the diary shows an indicator of the severity of the symptom 81 (for example in the form of an icon) and information identifying the symptom 82 (for example the date and allergy). Moreover, for each symptom detected and recorded a modification key 83 is shown, this key, once selected, allowing the subjective and objective information relating to that symptom to be modified, as well as a delete key 84 which allows the detection of that symptom to be deleted from the therapeutic diary.

The diary screen 80 furthermore may also optionally comprise one or more of the following items of information:
- date and current time; and
- time lapsed since the last detection of a respiratory difficulty symptom (for example in number of days).

The diary screen 80 furthermore may also optionally comprise one or more of the following keys:
- key for recording the detection of a symptom 85 which, similar to the key 14 of the main screen 10 and the key 45 of the menu screen 40, allows the recording graphical interface 50 to be opened; and
- key for accessing the map 86, which, similar to the key 15 of the main screen 10 and the key 42 of the menu screen 40, allows the map 71 to be opened.

The software component preferably is configured to allow the person to send in a simple manner the therapeutic diary stored on the mobile device 1 to her/his doctor. For this purpose, the diary screen preferably also comprises a send key 87 which, when selected by the person, causes the mobile device 1 to transmit the therapeutic diary to a predefined recipient, for example in the form of an e-mail to the e-mail address of her/his doctor. In this way, the doctor advantageously receives a complete and reliable therapeutic diary which allows the most suitable therapy to be established.

According to an advantageous variant, the software component is an app which may be downloaded onto the mobile device 1 by the person using the mobile device 1. For example, the software component may be an app developed in a .net 4.0 Microsoft environment using MS SQLServer as DBMS (Data Base Management System). Preferably, the software architecture also envisages the adoption of an ORM (Object-Relational Mapping) layer which provides, by means of an object-oriented interface, all the services relating to data persistence, while extracting at the same time the implementation characteristics of the specific DBMS used. The use of an ORM layer also ensures a high degree of portability with regard to the DBMS technology used (if the DBMS is changed the routines implementing the persistence layer do not have to be rewritten), manages the conflicts and the competition during access to the data being modified, and offers data caching mechanisms for improving the performance features of the system. For example, in order to develop the app, the known mobile cross-platform framework PhoneGap may be used, this allowing native applications to be developed by means of the use of web technologies such as HTML, CSS and JavaScript.

Advantageously, therefore, by interacting via the graphical interface of her/his mobile device (which the person always has with her/him), the person may therefore easily record on her/his mobile device the subjective information and objective information relating to each respiratory difficulty event as soon as the event has finished. The information relating to each difficulty event is therefore advantageously complete and reliable. The recording of one difficulty event after another on the mobile device therefore advantageously results in the automatic compilation of a therapeutic diary which is complete and reliable. Furthermore, the information relating to each detected difficulty symptom of the person may be at least partly shared with other persons suffering from the same disorder, so as to complete or correct the information given in the pollen bulletin.

## Claims

1. A method for providing information indicative of concentration of at least one allergen in the environment, said method comprising:
a) recording on a mobile device (1) information relating to detection of a respiratory difficulty symptom in a person, said recording comprising receiving from said person, via a graphical interface (50) of said mobile device (1), at least one subjective information relating to said detected respiratory difficulty symptom, said at least one subjective information comprising a probable cause of said detected respiratory difficulty symptom and an indication of the severity of said detected respiratory difficulty symptom, said recording further comprising automatically generating at least one objective information relating to said detected respiratory difficulty symptom, said at least one objective information comprising a place where said symptom was detected and a date and time at which said symptom was detected, said place and said date and time being automatically detected by said mobile device (1);
b) sharing on a sharing server (3) at least part of said information relating to said detected respiratory difficulty symptom and recorded on said mobile device (1), said at least part of said information relating to said detected respiratory difficulty symptom and recorded on said mobile device (1) comprising said place where said symptom was detected, said date and time at which said symptom was detected and said indication of said probable cause of said detected respiratory difficulty symptom; and
c) at a further mobile device (4a, 4b, 4c), receiving from said sharing server (3) said shared at least part of said information relating to said detected respiratory difficulty symptom and displaying said shared at least part of said information relating to said detected respiratory difficulty symptom on a map (71) shown by a display of said further mobile device (4a, 4b, 4c).

2. The method according to any of the preceding claims, wherein step a) also comprises locally storing said at least one objective information and said at least one subjective information relating to said detected respiratory difficulty symptom on said mobile device (1).

3. The method according to any of the preceding claims, wherein step b) comprises extracting signaling information relating to said detected respiratory difficulty symptom from said at least one objective information and said at least one subjective information and transmitting said signaling information to said sharing server (3) via a mobile radio network (2).

4. The method according to claim 3, wherein at step b) said signaling information also comprises said indication of the severity of said detected respiratory difficulty symptom.

5. The method according to any claim 3 or 4, wherein step b) comprises storing said signaling information on said sharing server (3) in an anonymous and non-traceable way.

6. The method according to any of claims 3 to 5, wherein step b) comprises storing said signaling information on said sharing server (3) for a finite time and deleted from said sharing server (3) upon expiry of said finite time.

7. The method according to any of claims 3 to 6, wherein step c) comprises showing on said map (71) an indicator symbol (72) corresponding to said signaling information.

8. The method according to claim 7, wherein step c) comprises showing said indicator symbol (72) on said map (71) substantially at said place where said symptom was detected as specified in said signaling information.

9. The method according to claim 8, wherein step c) comprises, upon manual selection of said indicator symbol (72), showing on said display of said further mobile device (4a, 4b, 4c) a pop-up menu containing said signaling information.

10. The method according to any one of the preceding claims, further comprising entering said at least one objective information and said at least one subjective information relating to said detected respiratory difficulty symptom in a therapeutic diary of said person, stored on said mobile device (1), said therapeutic diary comprising a temporally ordered list of respiratory difficulty symptoms detected by said person and recorded on said mobile device (1).

11. A communication system (100) for providing information indicative of concentration of at least one allergen in the environment, said communication system (100) comprising:
a) a mobile device (1) configured to record information relating to detection of a respiratory difficulty symptom in a person, said mobile device (1) being configured to receive from said person, via a graphical interface (50) of said mobile device (1), at least one subjective information relating to said detected respiratory difficulty symptom, said at least one subjective information comprising a probable cause of said detected respiratory difficulty symptom and/or an indication of the severity of said detected respiratory difficulty symptom, and to automatically generate at least one objective information relating to said detected respiratory difficulty symptom, said at least one objective information comprising a place where said symptom was detected and/or a date and time at which said symptom was detected, said place and said date and time being automatically detected by said mobile device (1);
b) a sharing server (3) configured to receive from said mobile device (1) at least part of said information relating to said detected respiratory difficulty symptom and recorded on said mobile device (1), said at least part of said information relating to said detected respiratory difficulty symptom and recorded on said mobile device (1) comprising said place where said symptom was detected, said date and time at which said symptom was detected and said indication of said probable cause of said detected respiratory difficulty symptom; and
c) a further mobile device (4a, 4b, 4c) configured to receive from said sharing server (3) said shared at least part of said information relating to said detected respiratory difficulty symptom and to display said shared at least part of said information relating to said detected respiratory difficulty symptom on a map (71) shown by a display of said further mobile device (4a, 4b, 4c).

12. A computer program product loadable in the memory of at least one computer, configured to perform the method according to any of claims 1 to 10, when said program is run by said at least one computer.

## Patentansprüche

1. Verfahren zum Bereitstellen von Informationen, die die Konzentration von mindestens einem Allergen in der Umgebung anzeigen, wobei das Verfahren aufweist:
a) Aufzeichnen von Informationen auf einer mobilen Einrichtung (1), die sich auf die Erfassung eines Symptoms einer Atemschwierigkeit bei einer Person beziehen, wobei das Aufzeichnen das Empfangen mindestens einer subjektiven Information, die sich auf das Symptom der Atemschwierigkeit bezieht von der Person über eine grafische Schnittstelle (50) der mobilen Einrichtung (1) aufweist, wobei die mindestens eine subjektive Information eine wahrscheinliche Ursache des detektierten Symptoms der Atemschwierigkeit und einen Hinweis auf den Schweregrad des detektierten Symptom der Atemschwierigkeit aufweist, wobei das Aufzeichnen des Weiteren aufweist das automatische Erzeugen von mindestens einer objektiven Information, die sich auf das detektierte Symptom der Atemschwierigkeit bezieht, wobei die mindestens eine objektive Information einen Ort, an dem das Symptom detektiert wurde und ein Datum und Zeit, zu welcher das Symptom detektiert wurde, aufweist, wobei der Ort, die Zeit und das Datum automatisch durch die mobile Einrichtung (1) detektiert werden;
b) Teilen auf einen Freigabeserver (3) zumindest einen Teil der Information, der sich auf das detektierte Symptom der Atemschwierigkeit bezieht und auf der mobilen Einrichtung (1) aufgezeichnet ist, wobei der mindestens eine Teil der Information, der sich auf das detektierte Symptom der Atemschwierigkeit bezieht und auf der mobilen Einrichtung (1) aufgezeichnet ist, den Ort an dem das Symptom detektiert wurde, das Datum und die Zeit zu dem das Symptom detektiert wurde und die wahrscheinliche Ursache des detektierten Symptom der Atemschwierigkeit aufweist; und
c) an einer weiteren mobilen Einrichtung (4a, 4b, 4c) empfangen von dem Freigabeserver (3) den geteilte mindesten einen Teil der Information, der sich auf das detektierte Symptom der Atemschwierigkeit bezieht und Anzeigen des geteilten mindestens einen Teils der Information, der sich auf das detektierte Symptom der Atemschwierigkeit beziehen auf einer Karte (71), die durch eine Anzeige der mobilen Einrichtungen (4a, 4b, 4c) gezeigt wird.

2. Verfahren nach einem der vorangehenden Ansprüche, wobei der Schritt a) auch das lokale Speichern auf der mobilen Einrichtung (1) der mindestens einen objektiven Information und der mindestens einen subjektiven Information aufweist, die sich auf das detektierte Symptom der Atemschwierigkeit beziehen.

3. Verfahren nach einem der vorangehenden Ansprüche, wobei der Schritt b) das Extrahieren von Signalisierungsinformationen bezüglich des detektierten Symptoms der Atemschwierigkeit aus der mindestens einen objektiven Information und der mindestens einen subjektiven Information und Übertragen der Signalisierungsinformation an den Freigabeserver (3) über ein mobiles Funknetzwerk (2) aufweist.

4. Verfahren nach Anspruch 3, wobei in Schritt b) die Signalisierungsinformation auch die Angabe des Schweregrades des detektierten Symptoms der Atemschwierigkeit aufweist.

5. Verfahren nach einem der Ansprüche 3 oder 4, wobei Schritt b) das Speichern der Signalisierungsinformation auf dem Freigabeserver (3) in einer anonymen und nicht rückverfolgbaren Weise aufweist.

6. Verfahren nach einem der Ansprüche 3 bis 5, wobei Schritt b) das Speichern der Signalisierungsinformation auf dem Freigabeserver (3) für eine endliche Zeit und das Löschen von dem Freigabeserver (3) nach dem Ablauf der endlichen Zeit aufweist.

7. Verfahren nach einem der Ansprüche 3 bis 6, wobei Schritt c) das Anzeigen auf der Karte (71) eines Indikatorsymbols (72), das der Signalisierungsinformation entspricht aufweist.

8. Verfahren nach Anspruch 7, wobei Schritt c) das Anzeigen des Indikatorsymbols (72) auf der Karte (71) im Wesentlichen an der Stelle aufweist, an der das Symptom wie in der Signalisierungsinformation angegeben, detektiert wurde.

9. Verfahren nach Anspruch 8, wobei Schritt c) aufweist bei manueller Auswahl des Indikatorsymbols (72), das Zeigen auf dem Display auf der Anzeige der weiteren mobilen Einrichtung (4a, 4b, 4c) eines Pop-Up Menüs, das die Signalisierungsinformation enthält.

10. Verfahren nach einem der vorangehenden Ansprüche des Weiteren aufweisend: Eingeben der mindestens einen objektiven Information und der mindestens einen subjektiven Information, die sich auf das detektierte Symptom der Atemschwierigkeit beziehen in ein therapeutisches Tagebuch der Person, das auf der mobilen Einrichtung (1) gespeichert ist, wobei das therapeutische Tagebuch eine zeitlich angeordnete Liste von Symptom der Atemschwierigkeit aufweist, die von der Person detektiert und auf der mobilen Einrichtung (1) aufgezeichnet wurden.

11. Kommunikationssystem (100) zum Bereitstellen von Information, die eine Konzentration von mindestens einem Allergen in der Umgebung anzeigen, wobei das Kommunikationssystem (100) aufweist:
a) eine mobile Einrichtung (1), die eingerichtet ist zum Aufzeichnen von Informationen, die sich auf die Erfassung eines Symptoms einer Atemschwierigkeit bei einer Person beziehen, wobei die mobile Einrichtung (1) eingerichtet ist mindestens eine subjektive Information, die sich auf das Symptom der Atemschwierigkeit bezieht von der Person über eine grafische Schnittstelle (50) zu erhalten, wobei die mindestens eine subjektive Information eine wahrscheinliche Ursache des detektierten Symptoms der Atemschwierigkeit und/oder einen Hinweis auf den Schweregrad des detektierten Symptom der Atemschwierigkeit aufweist, und zu automatischen Erzeugen von mindestens einer objektiven Information, die sich auf das detektierte Symptom der Atemschwierigkeit bezieht, wobei die mindestens eine objektive Information einen Ort, an dem das Symptom detektiert wurde und/oder ein Datum und Zeit, zu welcher das Symptom detektiert wurde, aufweist, wobei der Ort, die Zeit und das Datum automatisch durch die mobile Einrichtung (1) detektiert werden;
b) einen Freigabeserver (3), der eingerichtet ist um von der mobilen Einrichtung (1) zumindest einen Teil der Information, der sich auf das detektierte Symptom der Atemschwierigkeit bezieht und auf der mobilen Einrichtung (1) aufgezeichnet ist zu erhalten, wobei der mindestens eine Teil der Information, der sich auf das detektierte Symptom der Atemschwierigkeit bezieht und auf der mobilen Einrichtung (1) aufgezeichnet ist, den Ort an dem das Symptom detektiert wurde, das Datum und die Zeit zu dem das Symptom detektiert wurde und die wahrscheinliche Ursache des detektierten Symptom der Atemschwierigkeit aufweist; und
c) eine weitere mobile Einrichtung (4a, 4b, 4c) die eingerichtet ist um von dem Freigabeserver (3) den geteilte mindesten einen Teil der Information, der sich auf das detektierte Symptom der Atemschwierigkeit bezieht zu empfangen und um den geteilten mindestens einen Teils der Information, der sich auf das detektierte Symptom der Atemschwierigkeit beziehen auf einer Karte (71) anzuzeigen, die durch eine Anzeige der mobilen Einrichtungen (4a, 4b, 4c) angezeigt wird.

12. Computerprogrammprodukt, das in den Speicher mindestens eines Computers geladen werden kann und konfiguriert ist, dass es das Verfahren nach einem der Ansprüche 1 bis 10 durchführt, wenn das Programm von mindestens einem Computer ausgeführt wird.

## Revendications

1. Procédé permettant de fournir des informations indicatives sur la concentration d'au moins un allergène dans l'environnement, ledit procédé comprenant les étapes ci-dessous consistant à :
a) enregistrer, sur un dispositif mobile (1), des informations connexes à la détection d'un symptôme de difficulté respiratoire chez une personne, ladite étape d'enregistrement comprenant l'étape consistant à recevoir, en provenance de ladite personne, par l'intermédiaire d'une interface graphique (50) dudit dispositif mobile (1), au moins une information subjective connexe audit symptôme de difficulté respiratoire détecté, ladite au moins une information subjective comprenant une cause probable dudit symptôme de difficulté respiratoire détecté et une indication de la gravité dudit symptôme de difficulté respiratoire détecté, ladite étape d'enregistrement comprenant en outre l'étape consistant à générer automatiquement au moins une information objective connexe audit symptôme de difficulté respiratoire détecté, ladite au moins une information objective comprenant un site où ledit symptôme a été détecté et une date et heure auxquelles ledit symptôme a été détecté, ledit site et lesdites date et heure étant automatiquement détectés par ledit dispositif mobile (1) ;
b) partager, sur un serveur de partage (3), au moins une partie desdites informations connexes audit symptôme de difficulté respiratoire détecté et qui sont enregistrées sur ledit dispositif mobile (1), ladite au moins une partie desdites informations connexes audit symptôme de difficulté respiratoire détecté et qui sont enregistrées sur ledit dispositif mobile (1) comprenant ledit site où ledit symptôme a été détecté, lesdites date et heure auxquelles ledit symptôme a été détecté et ladite indication de ladite cause probable dudit symptôme de difficulté respiratoire détecté ; et
c) au niveau d'un dispositif mobile supplémentaire (4a, 4b, 4c), recevoir, en provenance dudit serveur de partage (3), ladite au moins partie partagée desdites informations connexes audit symptôme de difficulté respiratoire détecté, et afficher ladite au moins partie partagée desdites informations connexes audit symptôme de difficulté respiratoire détecté sur une carte (71) affichée par un écran d'affichage dudit dispositif mobile supplémentaire (4a, 4b, 4c).

2. Procédé selon la revendication précédente, dans lequel l'étape a) comprend également l'étape consistant à stocker localement ladite au moins une information objective et ladite au moins une information subjective, connexes audit symptôme de difficulté respiratoire détecté, sur ledit dispositif mobile (1).

3. Procédé selon l'une quelconque des revendications précédentes, dans lequel l'étape b) comprend l'étape consistant à extraire des informations de signalisation, connexes audit symptôme de difficulté respiratoire détecté, de ladite au moins une information objective et de ladite au moins une information subjective, et l'étape consistant à transmettre lesdites informations de signalisation audit serveur de partage (3) par l'intermédiaire d'un réseau radio mobile (2).

4. Procédé selon la revendication 3, dans lequel, à l'étape b), lesdites informations de signalisation comprennent également ladite indication de la gravité dudit symptôme de difficulté respiratoire détecté.

5. Procédé selon l'une quelconque des revendications 3 et 4, dans lequel l'étape b) comprend l'étape consistant à stocker lesdites informations de signalisation sur ledit serveur de partage (3) de manière anonyme et non traçable.

6. Procédé selon l'une quelconque des revendications 3 à 5, dans lequel l'étape b) comprend l'étape consistant à stocker lesdites informations de signalisation sur ledit serveur de partage (3) pendant un temps limité, et dans lequel lesdites informations sont supprimées dudit serveur de partage (3) à l'expiration dudit temps limité.

7. Procédé selon l'une quelconque des revendications 3 à 6, dans lequel l'étape c) comprend l'étape consistant à afficher, sur ladite carte (71), un symbole indicateur (72) correspondant auxdites informations de signalisation.

8. Procédé selon la revendication 7, dans lequel l'étape c) comprend l'étape consistant à afficher ledit symbole indicateur (72) sur ladite carte (71) sensiblement au niveau dudit site où ledit symptôme a été détecté, tel que spécifié dans lesdites informations de signalisation.

9. Procédé selon la revendication 8, dans lequel l'étape c) comprend l'étape consistant à, suite à une sélection manuelle dudit symbole indicateur (72), afficher, sur ledit écran d'affichage dudit dispositif mobile supplémentaire (4a, 4b, 4c), un menu contextuel contenant lesdites informations de signalisation.

10. Procédé selon l'une quelconque des revendications précédentes, comprenant en outre l'étape consistant à saisir ladite au moins une information objective et ladite au moins une information subjective, connexes audit symptôme de difficulté respiratoire détecté, dans un journal thérapeutique de ladite personne, stocké sur ledit dispositif mobile (1), ledit journal thérapeutique comprenant une liste ordonnée dans le temps de symptômes de difficulté respiratoire détectés par ladite personne et enregistrés sur ledit dispositif mobile (1).

11. Système de communication (100) destiné à fournir des informations indicatives sur la concentration d'au moins un allergène dans l'environnement, ledit système de communication (100) comprenant :
a) un dispositif mobile (1) configuré de manière à enregistrer des informations connexes à la détection d'un symptôme de difficulté respiratoire chez une personne, ledit dispositif mobile (1) étant configuré de manière à recevoir, en provenance de ladite personne, par l'intermédiaire d'une interface graphique (50) dudit dispositif mobile (1), au moins une information subjective connexe audit symptôme de difficulté respiratoire détecté, ladite au moins une information subjective comprenant une cause probable dudit symptôme de difficulté respiratoire détecté et/ou une indication de la gravité dudit symptôme de difficulté respiratoire détecté, et à générer automatiquement au moins une information objective connexe audit symptôme de difficulté respiratoire détecté, ladite au moins une information objective comprenant un site où ledit symptôme a été détecté et/ou une date et heure auxquelles ledit symptôme a été détecté, ledit site et lesdites date et heure étant automatiquement détectés par ledit dispositif mobile (1) ;
b) un serveur de partage (3) configuré de manière à recevoir, en provenance dudit dispositif mobile (1), au moins une partie desdites informations connexes audit symptôme de difficulté respiratoire détecté et qui sont enregistrées sur ledit dispositif mobile (1), ladite au moins une partie desdites informations connexes audit symptôme de difficulté respiratoire détecté et qui sont enregistrées sur ledit dispositif mobile (1) comprenant ledit site où ledit symptôme a été détecté, lesdites date et heure auxquelles ledit symptôme a été détecté et ladite indication de ladite cause probable dudit symptôme de difficulté respiratoire détecté ; et
c) un dispositif mobile supplémentaire (4a, 4b, 4c) configuré de manière à recevoir, en provenance dudit serveur de partage (3), ladite au moins partie partagée desdites informations connexes audit symptôme de difficulté respiratoire détecté, et à afficher ladite au moins partie partagée desdites informations connexes audit symptôme de difficulté respiratoire détecté sur une carte (71) affichée par un écran d'affichage dudit dispositif mobile supplémentaire (4a, 4b, 4c).

12. Produit-programme informatique pouvant être chargé dans la mémoire d'au moins un ordinateur, configuré de manière à mettre en œuvre le procédé selon l'une quelconque des revendications 1 à 10, lorsque ledit produit-programme informatique est exécuté par ledit au moins un ordinateur.
